# EUROPÄISCHE PATENTSCHRIFT

(11) **EP 3 243 536 B1**
(45) Veröffentlichungstag und Bekanntmachung des Hinweises auf die Patenterteilung: **29.06.2022**
(21) Anmeldenummer: 16169544.0
(22) Anmeldetag: 13.05.2016
(51) Int. Cl.: A61M 1/14, A61M 1/28, F04B 43/067

(54) **DIALYSEGERÄT MIT EINEM AKTOR UND EINEM HYDRAULIKSYSTEM**
MACHINE FOR DIALYSIS WITH AN ACTUATOR AND A HYDRAULIC SYSTEM
APPAREIL DE DIALYSE COMPRENANT UN ACTIONNEUR ET UN SYSTEME HYDRAULIQUE

(43) Veröffentlichungstag der Anmeldung: 15.11.2017
(73) Patentinhaber: Fresenius Medical Care Deutschland GmbH, 61352 Bad Homburg (DE)
(72) Erfinder: Hedmann, Frank, 97332 Volkach (DE); Sebesta, Sven, 91353 Hausen (DE)
(74) Vertreter: Herrmann, Uwe

(56) Entgegenhaltungen:
- DE-A1-102010 005 746
- DE-A1-102014 010 108
- DE-B- 1 053 316
- US-A- 3 291 055

## Beschreibung

Die Erfindung betrifft ein Dialysegerät mit einem Aktor und einem Hydrauliksystem zur Betätigung des Aktors gemäß dem Oberbegriff des Anspruchs 1.

In Dialysegeräten und beispielsweise in Peritonealdialysegeräten kommen oftmals hydraulische Aktorsysteme wie beispielsweise hydraulisch betriebene Pumpen zum Einsatz. Diese können beispielsweise dazu dienen, eine Dialyseflüssigkeit in einem Disposable zu fördern. Um die Gasfreiheit derartiger hydraulischer Systeme zu gewährleisten und um eine exakte Steuerung der Pumpe und somit Bilanzierung des Dialysefluids zu erreichen, ist im Stand der Technik bisweilen eine aufwändige konstruktive Gestaltung des hydraulischen Systems erforderlich, insbesondere dann, wenn eine Entgasung über das Disposable nicht erforderlich sein soll (was dann in vorteilhafter Weise zu konstruktiv einfacheren Disposables führt).

Die DE 10 2010 005 746 A1 offenbart ein Dialysegerät mit einem Pumpaktor und einem Hydrauliksystem, wobei das Hydrauliksystem einen Druckzylinder und eine vom Druckzylinder zum Aktor führende Druckleitung aufweist. Der Druckzylinder weist eine Mantelfläche und zwei Bodenflächen auf, wobei die Längsachse des Druckzylinders horizontal verläuft. Die DE 1 053 316 B offenbart eine Entlüftungsvorrichtung für eine hydraulisch, durch einen Kolben betätigte Membranpumpe, wobei der Kolben in einem Druckzylinder verschieblich angeordnet ist, dessen Längsachse horizontal verläuft. In der Mantelfläche des Druckzylinders befindet sich eine Entgasungsbohrung, die im normalen Betrieb von dem Kolben überfahren wird, um stets eine Entgasung zu bewirken.

Aufgabe der Erfindung ist es, ein Dialysegerät mit einem einfach konstruierten gattungsgemäßen Hydrauliksystem bereitzustellen, bei dem eine Entgasung über das Disposable entbehrlich ist.

Die Erfindung ist durch die Merkmale des Anspruchs 1 definiert.

Vor diesem Hintergrund betrifft die Erfindung ein Dialysegerät mit einem Aktor und einem Hydrauliksystem zur Betätigung des Aktors, wobei das Hydrauliksystem einen Druckzylinder und eine vom Druckzylinder zum Aktor führende Druckleitung aufweist, wobei der Druckzylinder Mantelwände und zwei Bodenflächen aufweist, und wobei der Druckzylinder einen Kolben aufweist, der in der Hauptachse des Druckzylinders verschoben werden kann, wobei der Druckzylinder so im Dialysegerät angeordnet ist, dass die Hauptachse in einem Neigungswinkel im Bereich von 5-45° zur Horizontalen steht, wobei die Druckleitung in der im abgesenkten Endbereich des Druckzylinders befindlichen abgesenkten Bodenfläche angeschlossen ist und wobei eine Entgasungsbohrung vorgesehen ist, die in einem nach oben zeigenden Mantelsegment des Druckzylinders angeordnet ist, wobei der Arbeitshub des Kolbens den Abschnitt des Druckzylinders zwischen der abgesenkten Bodenfläche und der Entgasungsbohrung einnimmt und wobei der Kolben in eine Entgasungsposition zum Entgasen der Hydraulikflüssigkeit verfahrbar ist, in der die Entgasungsbohrung in dem Abschnitt zwischen dem Kolben und der abgesenkten Bodenfläche liegt.

Der Kolben liegt dichtend an den Innenwänden des Druckzylinders an und trennt innerhalb des Druckzylinders eine Arbeits- von einer Ausgleichskammer. Die Arbeitskammer ist auf der abgesenkten und die Ausgleichskammer auf der angehobenen Seite des Kolbens angeordnet.

Der Druckzylinder weist eine Mantelfläche und zwei Bodenflächen auf, wobei die Positionsangaben *"abgesenkt"* und *"angehoben"* aus der Neigung der Hauptachse resultieren. Als die abgesenkte Bodenfläche ist die aufgrund der Neigung auf einem tieferen Niveau liegende Bodenfläche zu verstehen, und als die angehobene Bodenfläche die aufgrund der Neigung auf einem höheren Niveau liegende Bodenfläche. Die Begriffe des *"abgesenkten"* bzw. *"angehobenen"* Endbereichs bezeichnen den Bereich an oder nahe der jeweiligen Bodenfläche. Die Positionsangaben *"oben"*, *"unten"* und *"seitlich"* bezeichnen über den Umfang des Mantels verteilte Positionen ohne Berücksichtigung der Neigung der Hauptachse.

In einer Ausführungsform hat der Druckzylinder einen runden Querschnitt.

In einer Ausführungsform ist die Druckleitung an der abgesenkten Bodenfläche des Druckzylinders angeschlossen.

In einer Ausführungsform ist die Entgasungsbohrung an einem oberen Scheitelpunkt des Zylindermantels und/oder in der angehobenen Hälfte oder im angehobenen Drittel des Druckzylinders angeordnet.

Erfindungsgemäß beträgt der Neigungswinkel der Hauptachse 5-45°, vorzugsweise 10-30° und weiter vorzugsweise 15-25°.

In einer Ausführungsform ist in einem angehobenen Mantelsegment des Druckzylinders zusätzlich zur Entgasungsbohrung eine Ausgleichsöffnung angeordnet, wobei die Ausgleichsöffnung vorzugsweise näher an der angehobenen Bodenfläche des Druckzylinders liegt als die Entgasungsbohrung.

In einer Ausführungsform stehen die Entgasungs- und/oder die Ausgleichsöffnung mit einem Ausgleichsbehälter in Verbindung. Die Verbindung zwischen der Entgasungs- und/oder die Ausgleichsöffnung und dem Ausgleichsbehälter kann anhand einer Leitung erfolgen. In der Verbindung zwischen Entgasungs- und/oder die Ausgleichsöffnung und Ausgleichsbehälter sind vorzugsweise keine Ventile angeordnet.

Der Ausgleichsbehälter kann als Reservoir für die Hydraulikflüssigkeit dienen, die in der Arbeitskammer und gegebenenfalls auch in der Ausgleichskammer vorliegt. Entsprechend kann der Ausgleichsbehälter einen Zugang für dessen Befüllen mit Hydraulikflüssigkeit aufweisen. Der Fluss von Hydraulikflüssigkeit zwischen dem Druckzylinder und dem Ausgleichsbehälter kann durch die Entgasungs- und/oder die Ausgleichsöffnung erfolgen. In einer Ausführungsform weist der Ausgleichsbehälter eine Umgebungsöffnung auf, die den Innenraum des Ausgleichsbehälters mit der Umgebungsluft verbindet. An der Umgebungsöffnung bzw. in einer von der Umgebungsöffnung abführenden Leitung kann ein Ventil angeordnet sein.

In einer Ausführungsform weist die Druckleitung oder der abgesenkte Endbereich des Druckzylinders einen Zugang zu deren Befüllung mit Hydraulikflüssigkeit auf. Sofern kein Ausgleichsbehälter vorhanden ist, kann das System anhand eines derartigen Zugangs mit Hydraulikflüssigkeit befüllt werden.

In einer Ausführungsform ist eine Saugvorrichtung vorgesehen, die mit der Entgasungsbohrung oder dem Innenraum des Ausgleichsbehälters verbunden und ausgebildet ist, an der Entgasungsbohrung oder im Innenraum des Ausgleichsbehälters einen Unterdruck zu erzeugen. Mit einer derartigen Saugvorrichtung kann der Entgasungsvorgang der Hydraulikflüssigkeit unterstützt werden. Geeignete Saugvorrichtungen umfassen einfache mechanische Vorrichtungen wie beispielsweise eine Spritze, die vorzugsweise an der Entgasungsbohrung ansetzt. Weitere geeignete Saugvorrichtungen umfassen beispielsweise eine motorbetriebene Vakuumpumpe, die vorzugsweise mit dem Innenraum des Ausgleichsbehälters in Verbindung steht.

In einer Ausführungsform ist das Dialysegerät so ausgebildet, dass der Kolben anhand eines Schrittmotors und einer Spindel bewegt wird. Bei dem Schrittmotor handelt es sich vorzugsweise um einen Synchronmotor, der die Spindel durch ein gesteuertes, schrittweise rotierendes, elektromagnetisches Feld von Statorspulen um einen definierten Winkel (Schritt) oder ein Vielfaches davon drehen kann. Die Spindel kann ein Außengewinde aufweisen, um anhand der vom Schrittmotor erzeugten Drehbewegung eine translatorische Bewegung in der Achse des Druckzylinders zu erzeugen. Sie durchläuft vorzugsweise eine Bohrung am angehobenen Boden des Druckzylinders, die ein Innengewinde aufweisen kann.

In einer Ausführungsform ist ein vozugsweise rotatorischer Kodierer vorgesehen, um die Position der Spindel zu bestimmen. Geeignete Beispiele umfassen optische, magnetische und mechanische Kodierer mit entsprechenden Kontakten an der Spindel.

Der Schrittmotor und/oder der Kodierer sind vorzugsweise an der Außenseite des angehobenen Bodens des Druckzylinders angeordnet. Der Schrittmotor und der Kodierer stehen vorzugsweise mit der Steuereinheit des Geräts in Verbindung. So kann beispielsweise das Signal des Kodierers genützt werden, um den Schrittmotor zu steuern.

Durch einen derartigen Antrieb des Kolbens kann gegenüber einer alternativen Motorsteuerung eine exaktere Fluiddosierung erreicht werden und Sensoren wie beispielsweise Längensensoren für eine Pumpe entfallen.

In einer Ausführungsform ist am Druckzylinder oder an der Druckleitung ein Drucksensor angeordnet. Beispielsweise kann der Drucksensor im abgesenkten Endbereich bzw. am abgesenkten Boden des Druckzylinders angeordnet sein.

In einer Ausführungsform handelt es sich bei dem Dialysegerät um ein Peritonealdialysegerät.

In einer Ausführungsform handelt es sich bei dem Aktor um eine vorzugsweise disposableseitig angeordnete Pumpe, wobei die Druckleitung mit einer Pumpkalotte in Verbindung steht.

Weitere Einzelheiten und Vorteile der Erfindung ergeben sich aus den nachfolgend anhand der Figuren beschriebenen Ausführungsbeispielen. In den Figuren zeigen:
- Figur 1:: ein Hydrauliksystem eines Dialysegeräts gemäß einer ersten Ausführungsform der Erfindung;
- Figur 2:: ein Hydrauliksystem eines Dialysegeräts gemäß einer zweiten Ausführungsform der Erfindung,
- Figur 3:: ein Hydrauliksystem eines Dialysegeräts gemäß einer dritten Ausführungsform der Erfindung, und
- Figur 4:: eine Darstellung zur Funktionsweise derartiger Hydrauliksysteme.

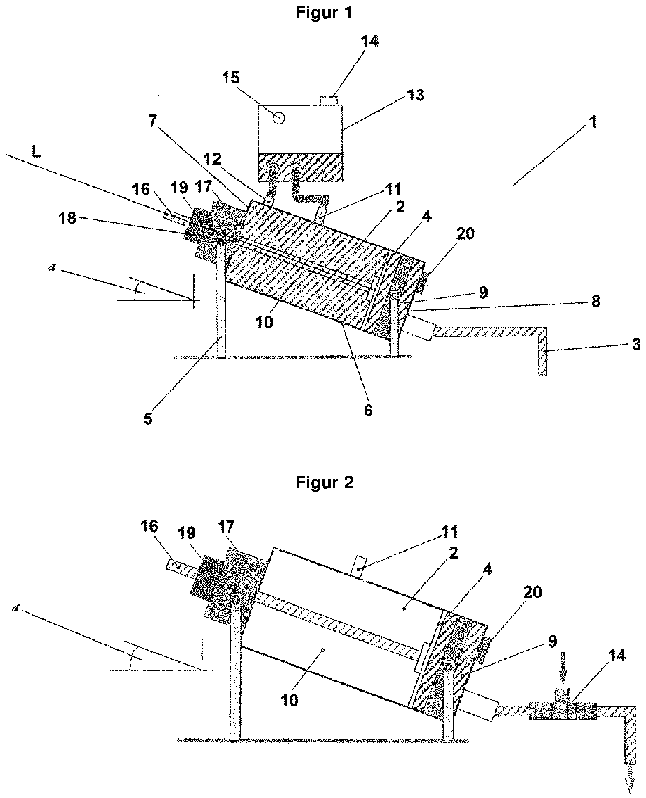

Das in Figur 1 dargestellte Hydrauliksystem ist generell mit dem Bezugszeichen 1 gekennzeichnet. Es umfasst einen Druckzylinder 2 und eine vom Druckzylinder 2 zur einer in der Figur nicht näher dargestellten Pumpkalotte eines in der Figur nicht näher dargestellten Disposables führende Druckleitung 3. Innerhalb des Druckzylinders 2 ist ein Kolben 4 angeordnet, der entlang der Hauptachse L des Druckzylinders 2 in Längsrichtung verschoben werden kann. Der Druckzylinder 2 ist innerhalb des Dialysegeräts mit schematisch dargestellten Haltemitteln 5 so gehalten, dass die Achse L in einem Winkel α von 20° zur Horizontalen geneigt ist. Der Druckzylinder 2 ist im Querschnitt rund und weist Mantelwände 6, eine durch die Neigung angehobene Bodenfläche 7 und eine durch die Neigung abgesenkte Bodenfläche 8 auf. Die Druckleitung 3 ist am abgesenkten Boden 8 angeschlossen. Der Kolben 4 liegt dichtend an der Innenseite der Mantelwände 6 an und trennt innerhalb des Druckzylinders 2 eine Arbeitskammer 9 von einer Ausgleichskammer 10, wobei die Arbeitskammer 9 auf der abgesenkten und die Ausgleichskammer 10 auf der angehobenen Seite des Kolbens 4 angeordnet ist.

Am oberen Scheitelpunkt der Mantelwände 6 ist etwa auf der angehobenen Hälfte des Druckzylinders 2 eine Entgasungsbohrung 11 vorgesehen. Zusätzlich ist am oberen Scheitelpunkt der Mantelwände 6 und weiter in der angehobenen Hälfte des Druckzylinders 2 eine Ausgleichsbohrung 12 vorgesehen. An beiden diesen Bohrungen sind Leitungen angeschlossen, die zu einem Ausgleichsbehälter 13 führen. Die Anschlüsse am Ausgleichsbehälter 13 stehen im Niveau über den Bohrungen 11 und 12. Der Ausgleichsbehälter 13 weist einen Zugang 14 zur Befüllung des Systems mit Hydraulikflüssigkeit sowie eine Umgebungsöffnung 15 auf. Die Umgebungsöffnung 15 verbindet den Innenraum des Ausgleichsbehälters 13 mit der Umgebungsluft und umfasst ein 2-Wege-Auslassventil.

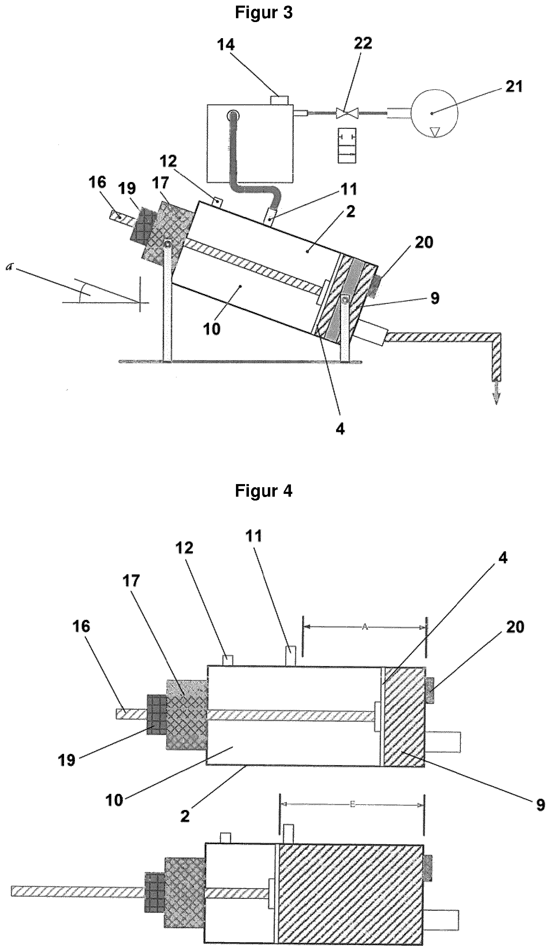

Da die Entgasungsbohrung 11 im normalen Pumpbetrieb (Erklärung siehe Figur 4) mit der gegenüber der Arbeitskammer 9 angehobenen Ausgleichskammer 10 verbunden ist, entleert sich der Ausgleichbehälter 13 in die Ausgleichskammer 10. Das Design macht es daher nötig, die Ausgleichsbohrung 12 einzufügen, um im Entgasungszyklus (Erklärung siehe Figur 4) das Flüssigkeitsvolumen in der Ausgleichskammer 10 während der Bewegung des Kolbens 4 in den Ausgleichbehälter 13 zu verdrängen.

Zur Steuerung der Verschiebung des Kolbens 4 entlang der Achse L sind eine Spindel 16 und ein Schrittmotor 17 vorgesehen. Die Spindel 16 verbindet den Kolben 4 mit dem Schrittmotor 17 und durchstößt den angehobenen Boden 7 des Druckzylinders 2 dabei durch eine Bohrung 18. Der Schrittmotor 17 ist ausgebildet, um eine Rotation der Spindel 16 zu bewirken. Die Spindel 16 weist ein Außengewinde auf, das mit einem Innengewinde in der Bohrung 18 derart zusammenwirkt, dass die Drehbewegung der Spindel 16 gleichzeitig eine translatorische Bewegung in der Achse L bewirkt. Direkt an Spindel 16 und am Motor 17 ist ferner ein Kodierer 19 vorgesehen, anhand dessen unter Zusammenwirken mit einem geeigneten Kontakt an der Spindel 16 die Position der Spindel 16 bestimmt werden kann. Der Schrittmotor 17 und der Kodierer 19 sind so mit einer in der Figur nicht näher dargestellten Steuereinheit verbunden, dass das Signal des Kodierers 19 zur Steuerung des Schrittmotors 17 genützt wird. Am abgesenkten Boden 8 des Druckzylinders 2 ist ein Drucksensor 20 angeordnet, der den Hydraulikdruck misst und ebenfalls mit der Steuereinheit des Geräts verbunden sein kann.

Der Drucksensor kann auch an einer anderen geeigneten Stelle angeordnet sein.

Das in der Figur 1 dargestellte Hydrauliksystem kann beispielsweise einen Teil eines Peritonealdialysegeräts darstellen, wobei die oben erwähnte Pumpkalotte an einem Disposable für den Ein- und Ablauf von Dialyseflüssigkeit wirkt.

Das in der Figur 2 dargestellte Hydrauliksystem unterscheidet sich von demjenigen gemäß Figur 1 dadurch, dass sowohl der Ausgleichsbehälter 13 als auch die Ausgleichsbohrung 12 fehlen. Die Entgasungsbohrung 11 ist mit der Umgebungsluft verbunden. Zur Verbesserung der Entgasung der Hydraulikflüssigkeit kann an der Entgasungsbohrung 11 eine in der Figur nicht näher dargestellte Saugvorrichtung in Form beispielsweise einer Spritze angelegt sein. Um das System trotz des fehlenden Ausgleichsbehälters 13 mit Hydraulikflüssigkeit befüllen zu können, ist an der Druckleitung 3 ein entsprechender Zugang 14 angeordnet. In der Ausgleichskammer 10 befindet sich (kompressible) Luft.

Dieses weiter vereinfachte System macht sich ein konstantes Flüssigkeitsvolumen zu Nutze, was es ermöglicht, den Ausgleichbehälter 13 zu entfernen. Das System kann einmalig über die Entgasungsbohrung 11 aktiv (z.B. mit einer Spritze) entlüftet werden. Der Zugang 14 ist vorgesehen, um das System zu befüllen.

Das in der Figur 3 dargestellte Hydrauliksystem unterscheidet sich von demjenigen gemäß Figur 1 dadurch, dass die Ausgleichsbohrung 12 nicht mit dem Ausgleichsbehälter 13 verbunden ist und dass am Ausgleichsbehälter 13 die Umgebungsöffnung 15 fehlt und der Ausgleichsbehälter stattdessen an eine motorbetriebene Vakuumpumpe 21 angeschlossen ist, die ausgebildet ist, im Innenraum des Ausgleichsbehälters 13 einen Unterdruck zu erzeugen. So kann der Entgasungsvorgang der Hydraulikflüssigkeit unterstützt werden. So wurde dem Aufbau gemäß der in Figur 2 dargestellten Ausführungsform eine Funktion hinzugefügt, um das Entgasen der Hydraulikflüssigkeit zu beschleunigen. Zwischen der Vakuumpumpe 21 und dem Ausgleichsbehälter ist ein Be- und Entlüftungsventil 22 angeordnet.

In Figur 4 wird die Funktionsweise eines erfindungsgemäßen Hydrauliksystems erklärt, die allen drei eben beschriebenen Systemen gemäß Figuren 1 bis 3 gemein ist. So wird der Kolben 4 innerhalb des Druckzylinders entlang der Achse L bewegt, um Hydraulikflüssigkeit aus der Arbeitskammer in die Druckleitung 3 und letztlich zur Pumpkalotte zu drücken. Der Arbeitshub des Kolbens 4 ist in der Figur mit dem Bezugszeichen A gekennzeichnet und nimmt den Abschnitt des Druckzylinders 2 zwischen dem abgesenkten Boden 9 und der Entgasungsbohrung 11 ein. Die Entgasungsposition zum Entgasen der Hydraulikflüssigkeit ist mit dem Bezugszeichen E gekennzeichnet. Sie entspricht einer Position des Kolbens 4 auf der angehobenen Seite der Entgasungsbohrung 11 bzw. zwischen der Entgasungsbohrung 11 und einer etwaigen Ausgleichsbohrung 12. Die Entgasungsbohrung 11 nimmt also das höchste Niveau des eigentlichen Arbeitsbereichs des Hydrauliksystems ein.

## Patentansprüche

1. Dialysegerät mit einem Aktor und einem Hydrauliksystem (1) zur Betätigung des Aktors, wobei das Hydrauliksystem (1) einen Druckzylinder (2) und eine vom Druckzylinder (2) zum Aktor führende Druckleitung (3) aufweist, wobei der Druckzylinder (2) Mantelwände (6) und zwei Bodenflächen (7, 8) aufweist, und wobei der Druckzylinder (2) einen Kolben (4) aufweist, der in der Hauptachse (L) des Druckzylinders (2) verschoben werden kann,
**dadurch gekennzeichnet,**
**dass** der Druckzylinder (2) so im Dialysegerät angeordnet ist, dass die Hauptachse (L) in einem Neigungswinkel (α) im Bereich von 5-45° zur Horizontalen steht, wobei die Druckleitung (3) in der im abgesenkten Endbereich des Druckzylinders (2) befindlichen abgesenkten Bodenfläche (8) angeschlossen ist und wobei eine Entgasungsbohrung (11) vorgesehen ist, die in einem nach oben zeigenden Mantelsegment des Druckzylinders (2) angeordnet ist, wobei der Arbeitshub des Kolbens (4) den Abschnitt des Druckzylinders (2) zwischen der abgesenkten Bodenfläche (8) und der Entgasungsbohrung (11) einnimmt und wobei der Kolben (4) in eine Entgasungsposition zum Entgasen der Hydraulikflüssigkeit verfahrbar ist, in der die Entgasungsbohrung (11) in dem Abschnitt zwischen dem Kolben (4) und der abgesenkten Bodenfläche (8) liegt.

2. Dialysegerät nach Anspruch 1, **dadurch gekennzeichnet, dass** die Entgasungsbohrung (11) an einem oberen Scheitelpunkt des Zylindermantels oder in der angehobenen Hälfte oder im angehobenen Drittel des Druckzylinders (2) angeordnet ist.

3. Dialysegerät nach einem der vorhergehenden Ansprüche, **dadurch gekennzeichnet, dass** der Neigungswinkel (α) der Hauptachse 10-30° oder 15-25° beträgt.

4. Dialysegerät nach einem der vorhergehenden Ansprüche, **dadurch gekennzeichnet, dass** in einem angehobenen Mantelsegment des Druckzylinders (2) zusätzlich zur Entgasungsbohrung (11) eine Ausgleichsöffnung (12) angeordnet ist, wobei die Ausgleichsöffnung (12) näher an der angehobenen Bodenfläche (7) des Druckzylinders (2) liegt als die Entgasungsbohrung (11).

5. Dialysegerät nach einem der vorhergehenden Ansprüche, **dadurch gekennzeichnet, dass** die Entgasungsbohrung (11) oder die Ausgleichsöffnung (12) oder sowohl die Entgasungsbohrung (11) als auch die Ausgleichsöffnung (12) mit einem Ausgleichsbehälter (13) in Verbindung stehen.

6. Dialysegerät nach Anspruch 5, **dadurch gekennzeichnet, dass** der Ausgleichsbehälter (13) eine Umgebungsöffnung (14) aufweist, die den Innenraum des Ausgleichsbehälters (13) mit der Umgebungsluft verbindet.

7. Dialysegerät nach einem der vorhergehenden Ansprüche, **dadurch gekennzeichnet, dass** die Druckleitung (3) oder der abgesenkte Endbereich des Druckzylinders (2) einen Zugang (14) zu deren Befüllung mit Hydraulikflüssigkeit aufweist.

8. Dialysegerät nach einem der vorhergehenden Ansprüche, **dadurch gekennzeichnet, dass** eine Saugvorrichtung (21) vorgesehen ist, die mit der Entgasungsbohrung (11) oder dem Innenraum des Ausgleichsbehälters (13) verbunden und ausgebildet ist, an der Entgasungsbohrung (11) oder im Innenraum des Ausgleichsbehälters (13) einen Unterdruck zu erzeugen.

9. Dialysegerät nach einem der vorhergehenden Ansprüche, **dadurch gekennzeichnet, dass** der Kolben (4) anhand eines Schrittmotors (17) und einer Spindel (16) bewegt wird.

10. Dialysegerät nach Anspruch 9, **dadurch gekennzeichnet, dass** ein rotatorischer Kodierer (19) vorgesehen ist, um die Position der Spindel (16) zu bestimmen.

## Claims

1. A dialyzer having an actuator and a hydraulic system (1) for actuating the actuator, the hydraulic system (1) having a pressure cylinder (2) and a pressure line (3) leading from the pressure cylinder (2) to the actuator, the pressure cylinder (2) having casing walls (6) and two base surfaces (8, 9), and the pressure cylinder (2) having a piston (4) which can be displaced in the main axis (L) of the pressure cylinder (2),
**characterized in that**
the pressure cylinder (2) is arranged in the dialyzer such that the main axis (L) is at an angle of inclination (a) to the horizontal in the range of 5-45°, the pressure line (3) being connected in the lowered base surface (8) located in the lowered end region of the pressure cylinder (2), and a degassing bore (11) being provided, which is arranged in an upward-facing casing segment of the pressure cylinder (2), the working stroke of the piston (4) occupying the portion of the pressure cylinder (2) between the lowered base surface (8) and the degassing bore (11), and the piston (4) being movable into a degassing position for degassing the hydraulic fluid, in which degassing position the degassing bore (11) is located in the portion between the piston (4) and the lowered base surface (8).

2. The dialyzer in accordance with claim 1, **characterized in that** the degassing bore is arranged at an upper apex of the cylinder casing or in the raised half or in the raised third of the pressure cylinder (2).

3. The dialyzer in accordance with one of the preceding claims, **characterized in that** the angle of inclination (a) of the main axis is 10-30° or 15-25°.

4. The dialyzer in accordance with one of the preceding claims, **characterized in that**, in addition to the degassing bore (11), an equalizing orifice (12) is arranged in a raised casing segment of the pressure cylinder (2), the equalizing orifice (12) being located closer to the raised base surface (7) of the pressure cylinder (2) than the degassing bore (11).

5. The dialyzer in accordance with one of the preceding claims, **characterized in that** the degassing bore (11) or the equalizing orifice (12) or both the degassing bore (11) and the equalizing orifice (12) are in connection with an equalizing vessel (13).

6. The dialyzer in accordance with claim 5, **characterized in that** the equalizing vessel (13) has an opening to the surroundings (14), which connects the internal space of the equalizing vessel (13) to the surrounding air.

7. The dialyzer in accordance with one of the preceding claims, **characterized in that** the pressure line (3) or the lowered end region of the pressure cylinder (2) has an access (14) for filling it with hydraulic fluid.

8. The dialyzer in accordance with one of the preceding claims, **characterized in that** a suction device (21) is provided, which is connected to the degassing bore (11) or the internal space of the equalizing vessel (13), and is configured to generate a negative pressure at the degassing bore (11) or in the internal space of the equalizing vessel (13).

9. The dialyzer in accordance with one of the preceding claims, **characterized in that** the piston (4) is moved with the aid of a stepper motor (17) and a spindle (16).

10. The dialyzer in accordance with claim 9, **characterized in that** a rotary encoder (19) is provided to determine the position of the spindle (16).

## Revendications

1. Appareil de dialyse avec un actionneur et un système hydraulique (1) pour l'actionnement de l'actionneur, dans lequel le système hydraulique (1) présente un cylindre de pression (2) et une conduite de pression (3) menant depuis le cylindre de pression (2) vers l'actionneur, dans lequel le cylindre de pression (2) présente des parois d'enveloppe (6) et deux surfaces de fond (7, 8), et dans lequel le cylindre de pression (2) présente un piston (4), qui peut être coulissé dans l'axe principal (L) du cylindre de pression (2),
**caractérisé en ce**
**que** le cylindre de pression (2) est disposé de telle sorte dans l'appareil de dialyse que l'axe principal (L) se trouve selon un angle d'inclinaison (a) dans la plage de 5 - 45° par rapport à l'horizontale, dans lequel la conduite de pression (3) est raccordée dans la surface de fond abaissée (8) se trouvant dans la zone d'extrémité abaissée du cylindre de pression (2) et dans lequel est prévu un alésage de dégazéification (11), qui est disposé dans un segment d'enveloppe, pointant vers le haut, du cylindre de pression (2), dans lequel la course de travail du piston (4) prend la place de la section du cylindre de pression (2) entre la surface de fond abaissée (8) et l'alésage de dégazéification (11) et dans lequel le piston (4) peut être déplacé dans une position de dégazéification pour dégazéifier le liquide hydraulique, dans laquelle l'alésage de dégazéification (11) se situe dans la section entre le piston (4) et la surface de fond abaissée (8).

2. Appareil de dialyse selon la revendication 1, **caractérisé en ce que** l'alésage de dégazéification (11) est disposé sur un point de sommet supérieur de l'enveloppe de cylindre ou dans la moitié relevée ou dans le tiers relevé du cylindre de pression (2).

3. Appareil de dialyse selon l'une quelconque des revendications précédentes, **caractérisé en ce que** l'angle d'inclinaison (a) de l'axe principal est de 10 - 30° ou de 15 - 25°.

4. Appareil de dialyse selon l'une quelconque des revendications précédentes, **caractérisé en ce qu'**une ouverture de compensation (12) est disposée dans un segment d'enveloppe relevé du cylindre de pression (2) en plus de l'alésage de dégazéification (11), dans lequel l'ouverture de compensation (12) se situe davantage à proximité de la surface de fond relevée (7) du cylindre de pression (2) que l'alésage de dégazéification (11).

5. Appareil de dialyse selon l'une quelconque des revendications précédentes, **caractérisé en ce que** l'alésage de dégazéification (11) ou l'ouverture de compensation (12) ou encore l'alésage de dégazéification (11) et l'ouverture de compensation (12) sont reliés à un contenant de compensation (13).

6. Appareil de dialyse selon la revendication 5, **caractérisé en ce que** le contenant de compensation (13) présente une ouverture vers l'environnement (14), qui relie l'espace intérieur du contenant de compensation (13) à l'air ambiant.

7. Appareil de dialyse selon l'une quelconque des revendications précédentes, **caractérisé en ce que** la conduite de pression (3) ou la zone d'extrémité abaissée du cylindre de pression (2) présente un accès (14) pour leur remplissage en liquide hydraulique.

8. Appareil de dialyse selon l'une quelconque des revendications précédentes, **caractérisé en ce qu'**un dispositif d'aspiration (21), qui est relié à l'alésage de dégazéification (11) ou à l'espace intérieur du contenant de compensation (13), est prévu et est réalisé pour produire une dépression sur l'alésage de dégazéification (11) ou dans l'espace intérieur du contenant de compensation (13).

9. Appareil de dialyse selon l'une quelconque des revendications précédentes, **caractérisé en ce que** le piston (4) est déplacé à l'aide d'un moteur pas à pas (17) et d'une broche (16).

10. Appareil de dialyse selon la revendication 9, **caractérisé en ce qu'**un encodeur (19) rotatif est prévu pour définir la position de la broche (16).
